# FASCICULE DE BREVET EUROPEEN

(11) **EP 3 400 047 B1**
(45) Date de publication et mention de la délivrance du brevet: **20.11.2019**
(21) Numéro de dépôt: 17711702.5
(22) Date de dépôt: 06.01.2017
(51) Int. Cl.: A61M 15/00, B05B 11/06

(54) **DISPOSITIF DE DISTRIBUTION NASALE DE POUDRE**
VORRICHTUNG ZUR NASALEN VERABREICHUNG EINES PULVERS
NASAL DELIVERY DEVICE FOR A POWDER

(30) Priorité: 07.01.2016 FR 1650118
(43) Date de publication de la demande: 14.11.2018
(73) Titulaire: Aptar France SAS, 27110 Le Neubourg (FR)
(72) Inventeur: BROUET, Guillaume, 76000 Rouen (FR); POULLAIN, Franck, 27400 La Haye Malherbe (FR); MICHELET, Olivier, 76100 Rouen (FR)
(74) Mandataire: CAPRI
(86) Numéro de dépôt international: PCT/FR2017/050040
(87) Numéro de publication internationale: WO 2017/118827

(56) Documents cités:
- WO-A1-02/45866
- WO-A1-99/46055
- WO-A1-2015/001281
- FR-A1- 2 952 042
- US-A1- 2014 283 820

## Description

La présente invention concerne un dispositif de distribution nasale de poudre.

Les dispositifs de distribution nasale de poudre sont bien connus. Ils comportent généralement un réservoir contenant une ou plusieurs doses de poudre, des moyens de distribution, et une tête de distribution nasale destinée à être insérée dans une narine d'un utilisateur, ladite tête de distribution nasale comportant un orifice de distribution. Les moyens de distribution comportent généralement une chasse d'air. Lorsque le dispositif de distribution est actionné, une dose de poudre est distribuée dans une narine de l'utilisateur.

Un inconvénient avec ces dispositifs de l'art antérieur concerne la distribution de la dose de poudre dans la narine.

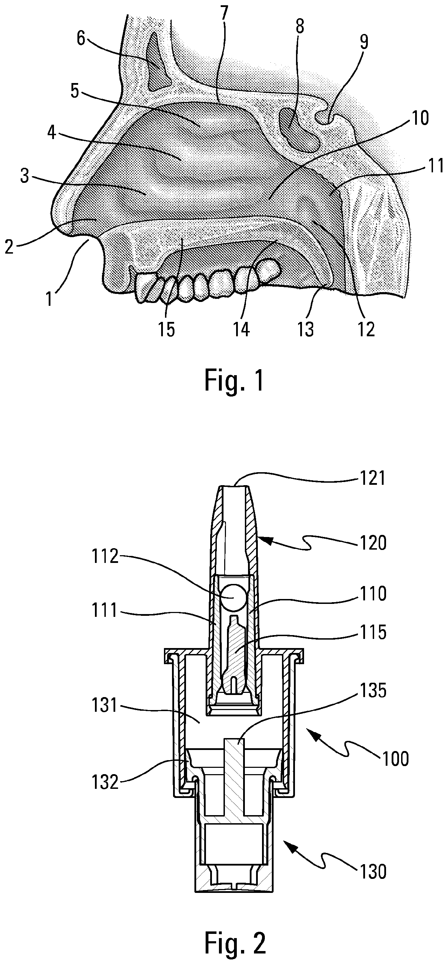

La figure 1 illustre schématiquement une telle narine. Celle-ci comprend l'orifice narinaire 1 (parfois appelé nez), la valve nasale 2, le cornet inférieur 3, le cornet intermédiaire 4 et le cornet supérieur 5, le sinus frontal 6, les éthmoïdes 7, le sinus sphénoïdal 8, la selle turcique 9, la choane 10, le cavum 11, la trompe d'Eustache 12, le voile du palais 13, le palais mou 14 et le palais dur 15.

La valve nasale possède une géométrie particulière. Elle s'étend sur environ 1 cm de profondeur et a une section longitudinale verticale d'environ 3 à 4 cm et une largeur d'environ 1 à 3 mm. Après le passage de la valve nasale, la cavité nasale comprend une plus grande cavité (environ 7 cm de hauteur sur 2 à 3 cm de largeur). Les cornets font face à la valve nasale. Au-dessus des cornets est situé le plafond de la cavité nasale, comprenant les ethmoïdes, le bulbe olfactif et le nerf olfactif.

Les dispositifs de distribution nasale n'étant pas ou peu invasifs, la poudre distribuée ne franchit généralement pas la valve nasale 2. Ainsi, de par l'anatomie de la valve nasale 2 et de l'emplacement protectif des cornets 3, 4, 5, la trajectoire axiale ou rectiligne des particules du spray de poudre ne permet pas d'atteindre le plafond de la cavité nasale, et notamment les ethmoïdes 7.

Les documents WO9946055, WO0245866 et WO2015001281 décrivent des dispositifs de l'état de la technique.

La présente invention a pour but de fournir un dispositif de distribution nasale de poudre qui ne reproduit pas les inconvénients susmentionnés.

La présente invention a aussi pour but de fournir un dispositif de distribution nasale de poudre qui améliore le pourcentage de la dose qui atteint les éthmoïdes.

La présente invention a également pour but de fournir un dispositif de distribution nasale de poudre qui est simple et peu coûteux à fabriquer et à assembler.

La présente a donc pour objet un dispositif de distribution nasale de poudre comportant un réservoir contenant au moins une dose de poudre, une tête de distribution nasale destinée à être insérée dans une narine d'un utilisateur, ladite tête de distribution nasale comportant un orifice de distribution, et une chasse d'air générant, lors de l'actionnement dudit dispositif de distribution nasale de poudre, un écoulement d'air comprimé pour distribuer une dose de poudre dans ladite narine à travers ledit orifice de distribution, ladite chasse d'air comprenant une chambre d'air et un piston coulissant de manière étanche dans ladite chambre d'air pour comprimer l'air contenu dans ladite chambre d'air, ledit dispositif de distribution nasale de poudre présentant les propriétés suivantes:
- une pression dudit écoulement d'air comprimé généré par ladite chasse d'air supérieure à 0,7 bar; et
- un volume de ladite chambre d'air supérieur à 1700 mm³.

Avantageusement, ladite pression est inférieure à 2 bar, notamment comprise entre 1 et 1,5 bar, de préférence environ 1,3 bar.

Avantageusement, ledit volume est inférieur à 3000 mm³, notamment compris entre 2000 et 2700 mm³, de préférence environ 2600 mm³.

Avantageusement, ladite dose de poudre est de 10 mg.

Avantageusement, la taille moyenne de particules de la dose de poudre est supérieure à 5 µm.

Avantageusement, ledit réservoir contient une seule dose de poudre, distribuée lors d'un seul actionnement dudit dispositif de distribution nasale de poudre.

Avantageusement, ledit dispositif de distribution nasale de poudre comporte une pluralité de réservoirs, chacun contenant une dose unique de poudre.

Ces caractéristiques et avantages et d'autres apparaîtront plus clairement au cours de la description détaillée suivante, faite en référence aux dessins joints, donnés à titre d'exemples non limitatifs, et sur lesquels :
- la figure 1 est une vue schématique d'une narine,
- la figure 2 est une vue schématique d'un dispositif de distribution nasale de poudre adapté pour la présente invention, et
- la figure 3 est un graphique illustrant les dépositions de poudre dans les différentes zones de la narine selon le volume de la chasse d'air et la pression générée par ladite chasse d'air.

Dans la description, les termes "axial" et "radial" se réfèrent en particulier à la direction longitudinale du dispositif représenté sur la figure 2. Les termes "proximal" et "distal" se réfèrent à l'orifice de distribution dudit dispositif.

L'invention s'applique plus particulièrement à des dispositifs du type unidose poudre tel que celui représenté sur la figure 2. Bien entendu, d'autres types de dispositifs de distribution nasale de poudre sont aussi envisageables.

Le dispositif 100 représenté sur la figure 2 comporte un réservoir 110 contenant une dose de poudre. Des dispositifs avec un réservoir contenant plus d'une dose sont possibles. De même, des dispositifs comportant plusieurs réservoirs contenant chacune une seule dose sont aussi possibles.

Une tête de distribution nasale 120 est assemblée sur ledit réservoir 110, ladite tête étant destinée à être insérée dans une narine d'un utilisateur. Ladite tête de distribution nasale comporte un orifice de distribution 121.

Le dispositif 100 comporte en outre une chasse d'air 130 générant, lors de l'actionnement dudit dispositif 100, un écoulement d'air comprimé pour distribuer une dose de poudre dans ladite narine à travers ledit orifice de distribution 121. Ladite chasse d'air comprend une chambre d'air 131 et un piston 132 coulissant de manière étanche dans ladite chambre d'air 131 pour comprimer l'air contenu dans ladite chambre d'air 131 et ainsi générer ledit écoulement d'air comprimé.

Dans l'exemple représenté sur la figure 2, le réservoir 110 est formé par un tube creux 111 ouvert à ses deux extrémités axiales, et fermé à son extrémité proximale par un élément de fermeture 112, tel qu'une bille, et fermé à son extrémité distale par un insert 115. Cet insert 115 comporte une extension axiale formant tige, et peut, lors de l'actionnement, coulisser dans ledit tube creux 111 pour chasser ledit élément de fermeture 112 hors de sa position de fermeture. Dans cet exemple, le piston 132 de la chasse d'air 130 est solidaire d'une projection axiale 135 qui s'étend en direction proximale, et qui, lors de l'actionnement, va se déplacer ensemble avec le piston 132 lors de la compression de l'air contenu dans la chambre d'air 131. Lorsque ladite projection 135 du piston 132 vient en contact avec ledit insert 115 du réservoir 110, une continuation du déplacement du piston 132 va provoquer le coulissement dudit insert 115 dans ledit tube creux 111 hors de sa position de fermeture. Ledit insert 115 va d'une part ouvrir le passage entre la chasse d'air 130 et le réservoir 110 et d'autre part provoquer l'expulsion de l'élément de fermeture 112. Ainsi, l'air comprimé dans la chambre d'air 131 va s'écouler dans ledit réservoir et entrainer la dose de poudre hors dudit réservoir en direction dudit orifice de distribution 121. Les documents WO9946055, WO0245866 et WO2015001281 décrivent des dispositifs de ce type. Bien entendu, d'autres types de dispositifs sont aussi possibles.

Selon l'invention, le dispositif de distribution nasale de poudre 100 présente les propriétés suivantes:
- une pression dudit écoulement d'air comprimé généré par ladite chasse d'air 130 supérieure à 0,7 bar, avantageusement inférieure à 2 bar, avantageusement comprise entre 1 et 1,5 bar, de préférence environ 1,3 bar; et
- un volume de ladite chambre d'air 131 supérieur à 1700 mm³, avantageusement inférieur à 3000 mm³, avantageusement compris entre 2000 et 2700 mm³, de préférence environ 2600 mm³.

Cette combinaison de pression et volume de la chasse d'air permet d'optimiser le taux de dépôt de poudre sur les éthmoïdes.

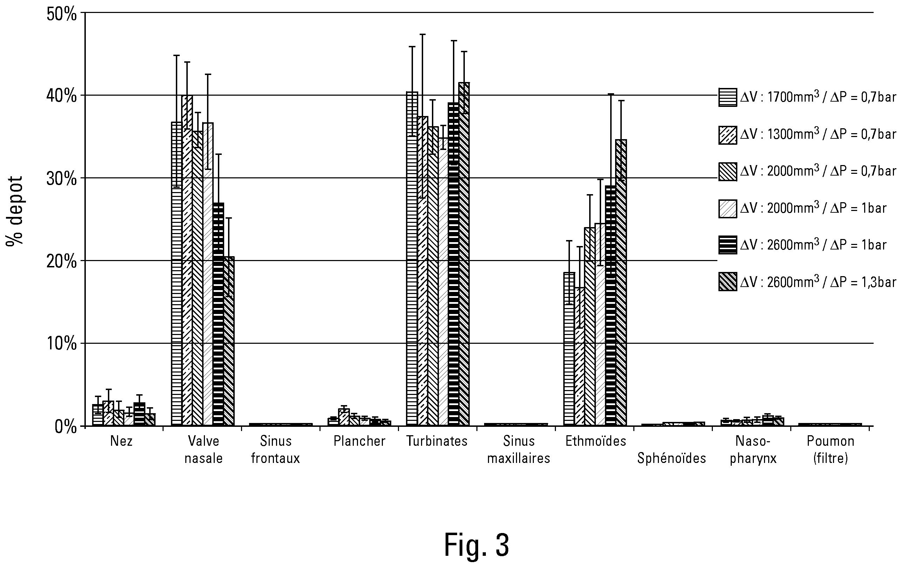

La figure 3 illustre des tests comparatifs qui démontrent, pour un même dispositif de distribution et pour une même dose de poudre de 10 mg:
- avec une pression identique de 0,7 bar, un volume de 2000 mm³ fournit un meilleur taux de déposition au niveau des éthmoïdes, comparé à des volumes des 1300 et 1700 mm³;
- avec un volume identique de 2000 mm³, une pression de 1 bar fournit un meilleur taux de déposition au niveau des éthmoïdes, comparé à une pression de 0,7 bar;
- avec un volume identique de 2600 mm³, une pression de 1,3 bar fournit un meilleur taux de déposition au niveau des éthmoïdes, comparé à une pression de 1 bar;
- le meilleur taux de déposition au niveau des éthmoïdes, environ 35%, est obtenu avec un volume de 2600 mm³ et une pression de 1,3 bar.

On peut noter que les valeurs de pression et de volume de la chasse d'air peuvent être dépendantes de la dose de poudre distribuée, et que les valeurs maximales peuvent être limitées par la gêne occasionnée à l'utilisateur et un actionnement du dispositif de distribution qui ne doit pas être trop difficile.

De manière connue pour une distribution nasale de poudre, il est souhaitable que la taille moyenne des particules de la dose de poudre soit supérieure à 5 µm.

La présente invention a été décrite en référence à un mode de réalisation avantageux, mais il est entendu qu'un homme du métier peut y apporter toutes modifications, sans sortir du cadre de la présente invention tel que défini par les revendications annexées.

## Revendications

1. Dispositif de distribution nasale de poudre (100) comportant un réservoir (110) contenant au moins une dose de poudre, une tête de distribution nasale (120) destinée à être insérée dans une narine d'un utilisateur, ladite tête de distribution nasale comportant un orifice de distribution (121), et une chasse d'air (130) générant, lors de l'actionnement dudit dispositif de distribution nasale de poudre (100), un écoulement d'air comprimé pour distribuer une dose de poudre dans ladite narine à travers ledit orifice de distribution (121), ladite chasse d'air comprenant une chambre d'air (131) et un piston (132) coulissant de manière étanche dans ladite chambre d'air (131) pour comprimer l'air contenu dans ladite chambre d'air (131), **caractérisé en ce que** ledit dispositif de distribution nasale de poudre (100) présente les propriétés suivantes:
- une pression dudit écoulement d'air comprimé généré par ladite chasse d'air (130) supérieure à 0,7 bar; et
- un volume de ladite chambre d'air (131) supérieur à 1700 mm³.

2. Dispositif selon la revendication 1, dans lequel ladite pression est inférieure à 2 bar.

3. Dispositif selon la revendication 1 ou 2, dans lequel ladite pression est comprise entre 1 et 1,5 bar.

4. Dispositif selon l'une quelconque des revendications précédentes, dans lequel ladite pression est environ 1,3 bar.

5. Dispositif selon l'une quelconque des revendications précédentes, dans lequel ledit volume est inférieur à 3000 mm³.

6. Dispositif selon l'une quelconque des revendications précédentes, dans lequel ledit volume est compris entre 2000 et 2700 mm³.

7. Dispositif selon l'une quelconque des revendications précédentes, dans lequel ledit volume est environ 2600 mm³.

8. Dispositif selon l'une quelconque des revendications précédentes, dans lequel ladite dose de poudre est de 10 mg.

9. Dispositif selon l'une quelconque des revendications précédentes, dans lequel la taille moyenne de particules de la dose de poudre est supérieure à 5 µm.

10. Dispositif selon l'une quelconque des revendications précédentes, dans lequel ledit réservoir (110) contient une seule dose de poudre, distribuée lors d'un seul actionnement dudit dispositif de distribution nasale de poudre (100).

11. Dispositif selon la revendication 10, dans lequel ledit dispositif de distribution nasale de poudre (100) comporte une pluralité de réservoirs (110), chacun contenant une dose unique de poudre.

## Patentansprüche

1. Pulverausgabevorrichtung (100) für die Nase, umfassend einen Behälter (110), der mindestens eine Pulverdosis enthält, einen Ausgabekopf (120) für die Nase, der dazu gedacht ist, in ein Nasenloch eines Benutzers eingeführt zu werden, wobei der Ausgabekopf für die Nase eine Ausgabeöffnung (121) umfasst, und eine Luftausstoßeinrichtung (130), die bei Betätigung der Pulverausgabevorrichtung (100) für die Nase einen komprimierten Luftstrom erzeugt, um eine Pulverdosis durch die Ausgabeöffnung (121) in das Nasenloch auszugeben, wobei die Luftausstoßeinrichtung eine Luftkammer (131) und einen Kolben (132), der dichtend in der Luftkammer (131) gleitet, umfasst, um die in der Luftkammer (131) enthaltene Luft zu komprimieren, **dadurch gekennzeichnet, dass** die Pulverausgabevorrichtung (100) für die Nase folgende Eigenschaften aufweist:
- einen Druck des komprimierten Luftstroms, der durch die Luftausstoßeinrichtung (130) erzeugt wird, von mehr als 0,7 bar; und
- ein Volumen der Luftkammer (131) von mehr als 1700 mm³.

2. Vorrichtung nach Anspruch 1, wobei der Druck unter 2 bar beträgt.

3. Vorrichtung nach Anspruch 1 oder 2, wobei der Druck zwischen 1 und 1,5 bar beträgt.

4. Vorrichtung nach einem der vorhergehenden Ansprüche, wobei der Druck etwa 1,3 bar beträgt.

5. Vorrichtung nach einem der vorhergehenden Ansprüche, wobei das Volumen unter 3000 mm³ beträgt.

6. Vorrichtung nach einem der vorhergehenden Ansprüche, wobei das Volumen zwischen 2000 und 2700 mm³ beträgt.

7. Vorrichtung nach einem der vorhergehenden Ansprüche, wobei das Volumen etwa 2600 mm³ beträgt.

8. Vorrichtung nach einem der vorhergehenden Ansprüche, wobei die Pulverdosis 10 mg beträgt.

9. Vorrichtung nach einem der vorhergehenden Ansprüche, wobei die mittlere Teilchengröße der Pulverdosis über 5 µm liegt.

10. Vorrichtung nach einem der vorhergehenden Ansprüche, wobei der Behälter (110) eine einzige Pulverdosis enthält, die bei einer einzigen Betätigung der Pulverausgabevorrichtung (100) für die Nase ausgegeben wird.

11. Vorrichtung nach Anspruch 10, wobei die Pulverausgabevorrichtung (100) für die Nase eine Vielzahl von Behältern (110) umfasst, die jeweils eine einzige Pulverdosis enthalten.

## Claims

1. A nasal powder dispenser device (100) comprising: a reservoir (110) containing at least one dose of powder; a nasal dispenser head (120) for inserting into a user's nostril, said nasal dispenser head including a dispenser orifice (121); and an air expeller (130) that, during actuation of said nasal powder dispenser device (100), generates a flow of compressed air so as to dispense a dose of powder into said nostril through said dispenser orifice (121), said air expeller comprising an air chamber (131) and a piston (132) that slides in airtight manner in said air chamber (131) so as to compress the air contained in said air chamber (131); the device being **characterized in that** said nasal powder dispenser device (100) presents the following properties:
- a pressure, of said flow of compressed air generated by said air expeller (130), that is higher than 0.7 bar; and
- a volume, of said air chamber (131), that is smaller than 1700 mm³.

2. A device according to claim 1, wherein said pressure is lower than 2 bar.

3. A device according to claim 1 or claim 2, wherein said pressure lies in the range 1 bar to 1.5 bar.

4. A device according to any preceding claim, wherein said pressure is about 1.3 bar.

5. A device according to any preceding claim, wherein said volume is smaller than 3000 mm³.

6. A device according to any preceding claim, wherein said volume lies in the range 2000 mm³ to 2700 mm³.

7. A device according to any preceding claim, wherein said volume is about 2600 mm³.

8. A device according to any preceding claim, wherein said dose of powder is 10 mg.

9. A device according to any preceding claim, wherein the average size of particles of the dose of powder is greater than 5 µm.

10. A device according to any preceding claim, wherein said reservoir (110) contains a single dose of powder for dispensing during a single actuation of said nasal powder dispenser device (100).

11. A device according to claim 10, wherein said nasal powder dispenser device (100) includes a plurality of reservoirs (110), each containing a single dose of powder.
